# EUROPEAN PATENT SPECIFICATION

(11) **EP 3 386 629 B1**
(45) Date of publication and mention of the grant of the patent: **10.08.2022**
(21) Application number: 16871879.9
(22) Date of filing: 12.12.2016
(51) Int. Cl.: B01J 19/12, A61L 2/08, A61L 2/10, C02F 1/30, C02F 1/32

(54) **FLUID TREATMENT SYSTEM**
FLÜSSIGKEITSBEHANDLUNGSSYSTEM
SYSTÈME DE TRAITEMENT DE FLUIDE

(30) Priority: 11.12.2015 US 201562386759 P
(43) Date of publication of application: 17.10.2018
(73) Proprietor: Trojan Technologies, London, ON N5V 4T7 (CA)
(72) Inventor: KUSTERMANS, Mark Adrian, Strathroy, Ontario N7G 3H3 (CA); SASGES, Michael, Victoria, British Columbia V8N 2N7 (CA); RENNIE, Robert, London, Ontario N6C 6B7 (CA)
(74) Representative: Sandri, Sandro
(86) International application number: PCT/CA2016/051456
(87) International publication number: WO 2017/096490

(56) References cited:
- EP-A1- 2 493 816
- WO-A1-95/13853
- US-A- 3 814 680
- US-A- 4 422 773
- US-A- 4 769 131
- US-A1- 2010 237 254

## Description

### BACKGROUND OF THE INVENTION

### FIELD OF THE INVENTION

The present invention relates to a device for fluid treatment.

### DESCRIPTION OF THE PRIOR ART

Fluid treatment systems are generally known in the art. Ultraviolet radiation is commonly used to disinfect fluids. UV inactivation has numerous advantages over other methods, including low operating costs and the elimination of chemicals.

For relatively clear fluids, simple systems employing ultraviolet light can be used to inactivate organisms in the fluid. For example, US 4,482,809 describes a simple array of UV lamps that may be immersed in a flowing liquid, and is ideally suited to treating drinking water or relatively clear wastewater.

For fluids with higher optical absorbance of UV radiation, very thin fluid layers may be used. For example, Baroin patented a device (FR1101958) employing an annular thin film of fluid with thickness "between 1.5 and 2mm" (See Figure 1 showing a cross-sectional view of a thin-film UV reactor. The thin annular gap, 11, is formed between the quartz sleeve, 6, and the outer reactor wall, 10). More complicated systems have been used to produce a thin film through centrifugal force, as in GB191007669. Thin-film systems have the inherent disadvantage caused by the nearly-stagnant flow in the boundary layer. This can result in highly over-treated fluid near the radiation source, and under-exposed fluid in the distant boundary layer.

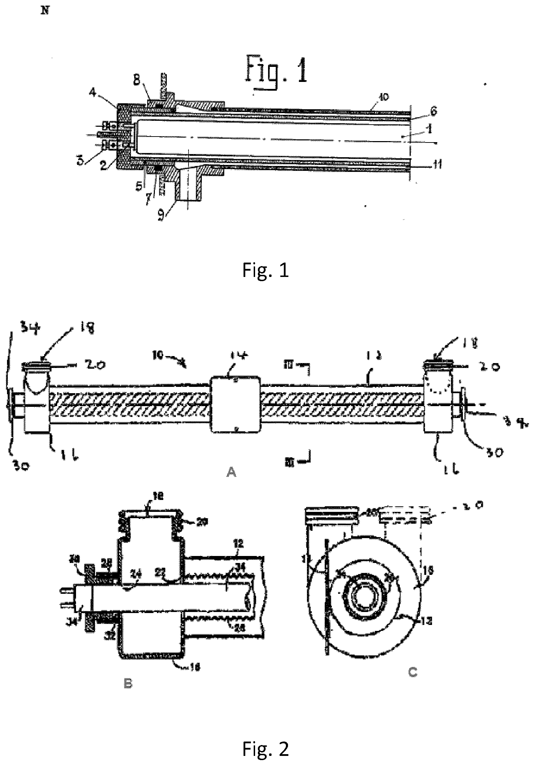

Another approach to treating strongly absorbing fluids is to induce mixing in the fluid in an attempt to ensure that all fluid elements are exposed to the UV radiation. US6916452 (Sure Pure) uses an annular flow path but attempts to induce swirling motion through a combination of the angle at which the fluid enters the annulus and a spiral groove in the outer wall of the reactor. (see Figure 2 showing A) the spiral fluting of the flow path, B) the inlet header, and C) the offset inlet and outlet (18 and 20) intended to induce swirl in the flowing liquid). A more complex approach is taken in US7695675 (Bayer) that claims both active, powered mixers and mixing based on fluid instability.

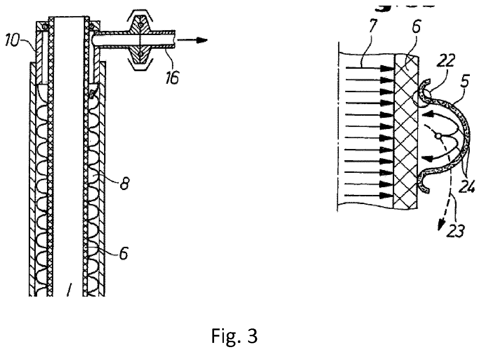

The system in US7695675 also claims a method to overcome the mixing problem by arranging a fluid conduit in a helical path around a central lamp (see Figure 3, where a helical fluid path is formed between a transparent (quartz) tube 6, and an outer fluted boundary, 5. The curvature (23) of this helix can give rise to Dean vortices denoted 24). Similar designs can be found in US5069782 and US5150705. Fluid flowing in this helical pathway can develop counter-rotating Dean Vortices that are intended to mix the fluid. However, the conduit has only a single curvature (the helix has only a single direction and pitch), so that the Dean Vortices, once formed, remain constant. An example is shown in Figure 3. Fluid at the center of the vortices will remain in the center, while the fluid at the edge of the vortices will rotate around these central elements. As a result, there is a stratification of doses rather than the intended mixing.

Others, as in US3814680 and US8653481, have arranged UV-transparent tubular fluid conduits around a central lamp, but with the tubing arranged in a primarily axial direction, with bends to redirect the fluid back along the opposite direction. Since alternate bent sections are at approximately 180° to each other, any resulting vortices are in opposite direction, but the peripheral and core areas are not changed. This system has the same drawback of stratified dose described above.

Still another approach is described in US4769131 and US4968437. Here the inventors claim two UV-irradiated flow conduits "helically coiled in a paired manner", incorporating a filter element between the two conduits. (See Figure 4, showing the serpentine double-path plus filter (not shown) of US4769131). A configuration that is described, but not claimed, shows two serpentine paths, each wrapping half-way around a central lamp, again with a filter element between the two paths. While this design incorporates a non-zero curvature as shown in Prior Art 3-B, which could induce vortexes, the subsequent reversal of direction shown in Prior Art 3-C has zero radius, and will not induce vortices.

Static mixers are a well-known and effective way to ensure good mixing in a fluid flow. These systems function by splitting the upstream flow into two or more paths and then rotating or twisting these paths. This process is repeated with subsequent mixers placed at an angle, typically 90° to the first, so that the fluid is repeatedly split and rotated. This approach is described in US6586172 (Iatros). Static mixers have the drawback that they act as an obstruction in the fluid flow and may become fouled with constituents of the flow. They also block the penetration of UV into the fluid conduit, which is a particularly important issue for strongly absorbing fluids.

Document WO 95/13853 A1 discloses a water purification unit that includes a cylindrical housing which has a water pipe and a water outlet. A sediment filter is connected to the pipe outside of the housing. On the inside of the housing, a stack helical coil of transparent tubing is held in place inside the housing. An ultraviolet lamp extends into the interior of the helical coil, for which power is obtained through a cord. The upper end of the coil passes downwardly through the unit as a tubing portion (50) that is connected to a water outlet pipe out of the housing base.

Document EP 2493816 A1 discloses a water purification apparatus, comprising an elongate UV source and a conduit for water to be purified formed of a UV transmissive material, wherein the conduit has an inlet and an outlet and is positioned so that part of it is wrapped around at least part of the UV source thereby to sterilise water within the conduit, further comprising reflective means for UV radiation to be reflected onto one or more parts of the conduit which extends beyond the part which is wrapped around the UV source.

Document US 4422773 A discloses an apparatus and method for thorough, non-invasive mixing of a flowing fluid stream, either continuous or segmented, and are embodied in a mixing conduit which comprises a plurality of interconnected bends, which may be in the form of arcuate coils or sections, successive arcuate sections being operable to establish secondary flow patterns in differing orientations within the flowing fluid stream, or individual segments thereof. Such arcuate sections may be disposed in a same plane, if formed in serpentine fashion, or in non-parallel, preferably orthogonal planes, if formed in a tortuous fashion. Also, the arcuate sections, or a series thereof may be formed in stacked fashion and located in essentially parallel planes.

Document US 3814680 A discloses an apparatus for purifying fluids having a bacterial population there which comprises: at least one standard 60 Hz. ultraviolet lamp; power supply means adapted to energize and drive said ultraviolet lamp. Said power supply means has a frequency of from I kHz. to 20 kHz. The apparatus further comprises a plurality of interconnected ultraviolet transmitting tubes arranged circumferentially around said lamp with the longitudinal axis of each tube being parallel to the longitudinal axis of said lamp, means for supplying said fluid to one of said tubes whereby said fluid enters one tube and flows along the length thereof and thereafter flows in opposite directions in succeeding tubes; and means for removing said fluid from one of said tubes.

Document US 4769131 A discloses a fluid purification system including an elongated ultraviolet radiation emitting tube and independent fluid flow-controlling conduits. Each conduit is transparent to allow ultraviolet light emitted by the tube to enter the conduit, and defines a continuous, and is helically wound closely about the tube to insure that fluid flow through the conduits is exposed to the ultraviolet light. The system includes a filter having inlet and outlet ports. An end of each conduit is connected to one of the inlet and outlet ports of the filter. The system thus exposes the fluid to ultraviolet radiation both before and after the fluid is filtered.

Document US 2010/237254 A1 discloses a treatment apparatus for at least partially disinfecting a fluid such as water comprising a pipe for conveying a flow of fluid to be treated, a series of ultraviolet (UV) LEDs for emission of the UV light into the fluid, and a control circuit for controlling operation of the LEDs. The control circuit is configured for pulsing the LEDs and the LEDs are arranged such that the fluid flows over a surface of each LED, as it is conveyed by the pipe in operation.

It would be desirable to have a UV-based fluid treatment device for opaque fluids that would achieve the targeted dividing and rotating of the static-mixer systems without the drawbacks of these designs.

### SUMMARY OF THE INVENTION

It is an object of the present invention to obviate or mitigate at least one of the above-mentioned disadvantages of the prior art.

It is another object of the present invention to provide a novel fluid treatment device suitable for UV-treatment of opaque fluid.

This is obtained by a device for fluid treatment having the features disclosed at claim 1. The dependent claims outline advantageous forms of embodiment of the device.

The device can be used to treat biological fluids and liquid foods including, but not limited to, whole blood and blood products including plasma, serum, vaccines, antibody solutions, cell culture media and constituents thereof, fermentation media, milk and milk products including whey, fruit juices, wine, cider and other liquids with strong absorbance or scattering of UV radiation.

In general, the a method not part of the present invention comprises the steps of irradiating a fluid with UV radiation between 200nm and 400nm while flowing the fluid in a fluid conduit that is at least partially transparent to said radiation, where the conduit has a section that is curved about a first axis, and a second section that is curved about a second axis that is not parallel to the first axis. Most preferably, these axes are at 90° to each other. Ideally, additional sections are included, each with an axis of curvature that is not parallel to that of the previous section. As a result of the fluid flowing in these curved sections, vortex motion may be induced in each curved section, and that motion is about an axis that is not parallel, and is nearly orthogonal to, the previous axis in the previous section. This has the result of repeatedly splitting and rotating the fluid so that it is well mixed and all the fluid receives the same amount of UV radiation.

Furthermore, since the fluid conduit of the present invention does not wrap repeatedly around a central axis of symmetry, the UV source may be placed at one axis of curvature and the conduit does not necessarily enclose the UV source. This allows the conduit to be removed in a direction perpendicular to said axis, rather than along the axis. This minimizes the required axial length required for replacement of the conduit or lamp, eliminating a shortcoming of the helical arrangement proposed by others.

Thus the present inventors have conceived of a novel fluid treatment device that can induce Dean Vortices in the flowing fluid, and then induce a new set of Dean Vortices at an angle to those in the first set. Each subsequent curved section can induce vortices at an angle to those in the last curved section. This reactor has the effect of repeatedly twisting and splitting the fluid flow, resulting in targeted mixing similar to that of static mixers without the necessity of utilizing physical mixers. This is also an improvement over helical tubing configurations that generate only a single set of vortices and do not split and mix the flow.

### BRIEF DESCRIPTION OF THE DRAWINGS

Embodiments of the present invention will be described with reference to the accompanying drawings, wherein like reference numerals denote like parts.
Figure 1 illustrates a thin-film annular reactor design from FR1101958 (prior art).
Figure 2 illustrates a prior art fluid treatment device, in which an annular fluid conduit is formed with fluted sides and offset inlet and outlet segments, in order to induce helical flow in the fluid. Figure 2B shows a detail of the same prior art system, with the inlet denoted 18. Figure 2C provides a detail of the orientation of the inlet and outlet, intended to induce swirl in the incoming fluid consistent with the spiral fluid conduit. This design has the effect of causing the flowing liquid to take on a helical path that induces counter-rotating vortices.
Figure 3 shows prior art from US 7420183 in which a helical flow path is formed between a transparent tube (6) and an outer fluted boundary, (5). There is only a single curvature radius and a single curvature axis

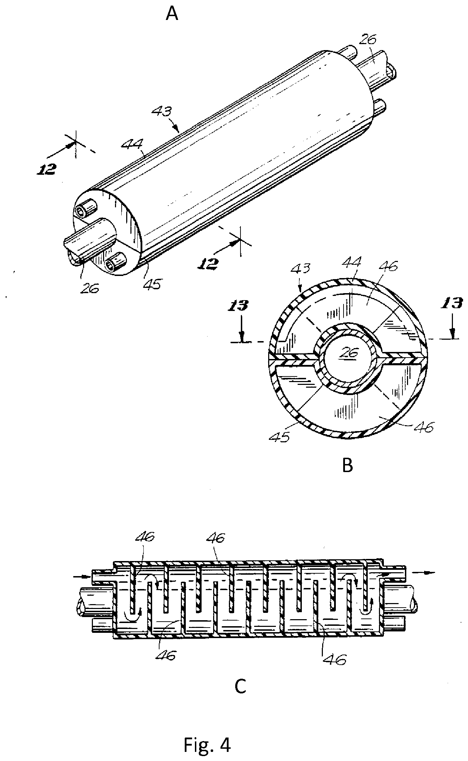

Figure 4 represents prior art from US 4769131 which does not use a tubular conduit and does not include alternating, non-zero radii of curvature for the fluid path. The square cross-section of the flow path is a hindrance to the formation of vortices, and the sharp, zero-radius curvature of the return portions will not induce vortices.

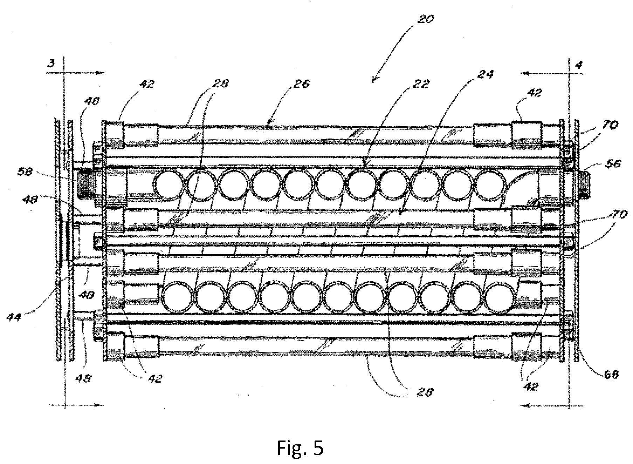

Figure 5 represents prior art from US 4798702, which arranges a UV-transmissive flow-conduit 22 into a helical shape irradiated by 6 V-lamps 24, 26, 28.
Figure 6 illustrates a reference embodiment of a fluid conduit arranged to achieve alternating curvatures. A UV emitter can be placed along the axis of curvature of one of these sections. In particular, this figure shows an UV-transmissive fluid conduit (1), arranged within an outer restraining shell (2). A UV emitter 3 is shown along one axis of curvature.
Figure 7 depicts the performance of a multi-pass reactor design over a wide range of fluid flow rates and optical properties.
Figure 8 depicts the performance of a very thin film reactor with a 1 mm water and built around the same lamp as that used in a preferred embodiment of the present fluid treatment device tested over a wide range of fluid flow rates and optical properties.
Figure 9 illustrates a helical spiral reactor constructed for comparison with a preferred embodiment of the present fluid treatment device.
Figure 10 shows the measured Fluence vs. 1/(Flow x Absorbance) for the Spiral (Helical) Reactor. The outer surface is covered with reflective foil to improve performance and protect the operator from transmitted UV.
Figure 11 is the computer generated fluid model of the fluid flow through curved section of the reactor.

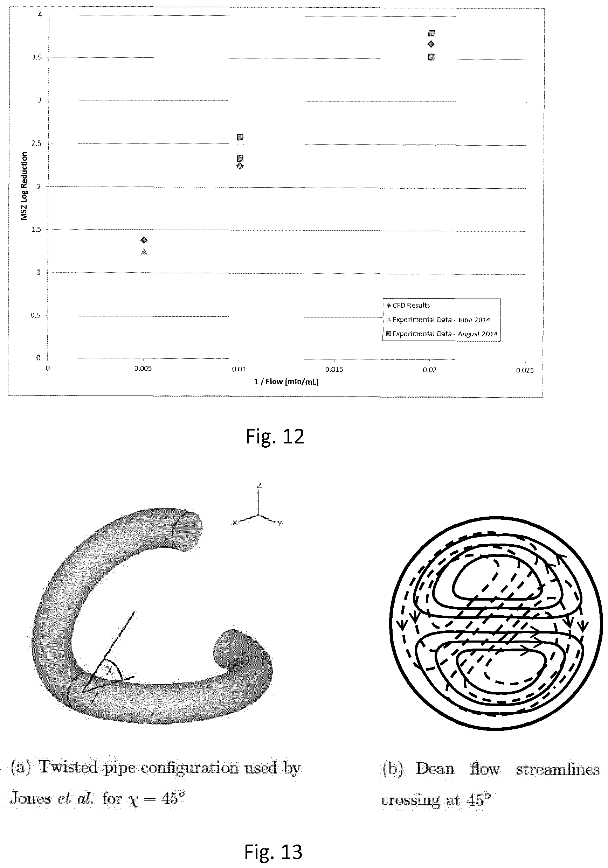

Figure 12 illustrates compared the results of the CFD model to experimental results
Figure 13 illustrates how curved sections of conduit, arranged in sequence, can set up vortices that are offset. This work is by Jones et al. In that work, the authors state that the ideal configuration with alternating curvatures cannot be achieved in reality, as they had not conceived of the present invention. They do not include or consider a UV source, and their analysis does not consider disinfection. Fig. 13(a) shows the twisted-conduit sections, while Fig. 13(b) shows the vortices formed by flow in the conduit.

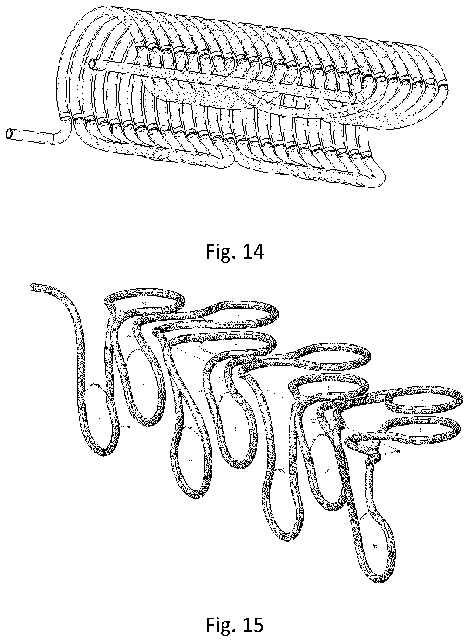

Figure 14 shows a convenient way to implement this alternating curvature. The fluid enters the conduit at the upper right, then curves -90 degrees and then travels in a direction along a curved outer retainer before curving in a direction roughly parallel to the axis of curvature of the outer retainer before following the curved retainer back in the opposite direction from the first, before again curving in a direction roughly parallel to the axis of curvature of the outer retainer but in opposite direction to the first pass. In this way, the tubing forms a large loop back to a point near the starting point. The second large loop is shifted axially with respect to the first, and this pattern is continued until the tubing has covered most of the area of the outer retainer in this region. At that point, a new looping pattern may be formed that is shifted axially with respect to the first, as shown in Figure 14. Each looping pattern can be formed by laying down a coil of tubing on a flat surface, then curving the flat surface into the retainer shape shown in Figure 14.
Figure 15 shows a configuration not comprised in the present invention that is particularly well adapted to planar light sources, such as flat arrays of Light Emitting Diodes. In this case the conduit has alternating curvature in order to induce alternating vortexes, but these alternating curvatures are in two planes that are arranged at 90 degrees to each other. Planar arrays of LEDs or other planar light sources (not shown) can be provide in each plane, each irradiating one of the set of curved segments.

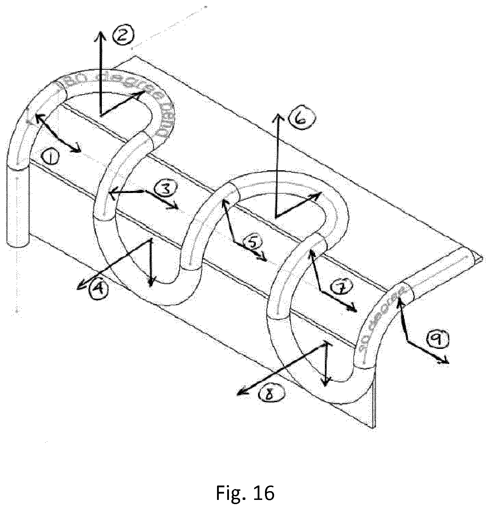

Figure 16 shows an embodiment according to the present invention, that has four curved sections in sequence, where the axes of the first and third section are parallel to each other, and orthogonal to the axes of the second and fourth sections. The axes of the second and fourth sections are orthogonal to each other. Sequential curved sections have axes of curvature that are orthogonal to each other: Curvature of the first section is orthogonal to that of the second; curvature of the second section is orthogonal to that of the third, etc. In particular, this figure shows a conduit configuration made up of repeated patterns with four curved sections in sequence. The odd- numbered axes (labeled 1, 3, 5, 7 and 9) are parallel to each other, and are orthogonal to those of the even-numbered sections (labelled 2, 4, 6 and 8). Sequential even-numbered axes are orthogonal to each other: axis 2 is orthogonal to axis 4; axis 4 is orthogonal to axis 6, etc.

### DETAILED DESCRIPTION OF THE PREFERRED EMBODIMENTS

Accordingly, in one of its aspects, the present invention provides a device for fluid treatment, comprising an UV-radiation source assembly, and a tubular fluid conduit that is at least partially transparent to radiation from the source assembly, wherein the tubular fluid conduit is configured to have repeated pattens of four curved sections in sequence, wherein each curved section is in a plane, where the axes of curvature of the first and third sections are parallel to each other and orthogonal to the axes of curvature of the second and fourth sections, and the axes of curvature of the second and fourth sections are orthogonal to each other, wherein said UV-radiation source assembly is placed at one axis of curvature of one of said sections and the conduit does not enclose the UV source.

Preferred embodiments of this device include the following features:
- the tubular conduit is circular in cross section;
- the UV-radiation source assembly emits in the wavelength range from 200nm to 400nm;
- the UV-radiation source assembly comprises a mercury-vapor lamp;
- the UV-radiation source assembly comprises Light Emitting Diodes; and/or
- the UV-radiation source assembly has power consumption between 5 watts and 500 watts.

The system preferably incorporates a central elongated mercury vapor lamp. It incorporates a fluid conduit constructed from tubing laid in a looping pattern that is also curved around the lamp axis, giving rise to two axes of curvature: one approximately aligned with the lamp axis, and the other perpendicular to the lamp axis.

The tubing can be made of a fluorinated material such as PTFE (polytetrafluoroethylene), FEP (Fluorinated Ethylene Propylene, for example Pharmafluor from St. Gobain), or THV (a polymer of tetrafluoroethylene, hexafluoropropylene and vinylidene fluoride). These materials are resistant to chemical attack, and are at least partially transparent to UV radiation.

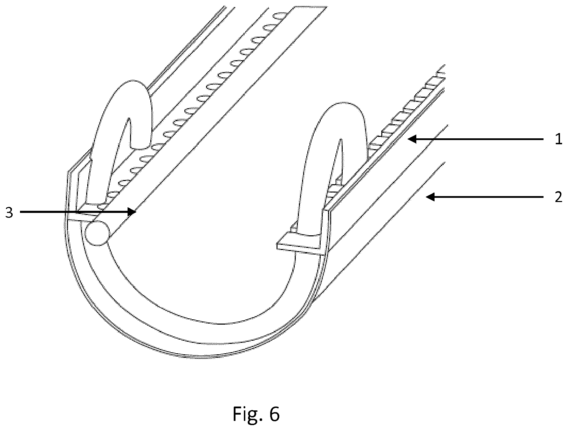

A simple way to achieve the desired arrangement is to lay the tubing in a coiled array along a flat surface, and then to press this coiled array into a curved shape. This configuration is shown in Figure 6, which includes two sequential coiled arrays as described above.

The same feature of alternating curvatures could also be made with tubing laid in a back-and-forth pattern with -270° return loops to bring the tubing back to the reverse direction. One curvature is in the return loop, the other curvature is in the section curved around the lamp.

The fluid conduit as described can be removed from the central lamp in a radial direction. This minimizes the space required for removing and replacing the fluid conduit or the lamp, which would otherwise require an axial length greater than two times the length of the lamp.

### PERFORMANCE OF A PREFERRED EMBODIMENT OF THE INVENTION

A system has been constructed as described in this document and tested over a wide range of fluid flow rates and optical properties. UV fluence (dose) delivery was evaluated using phage (virus) inoculated into fluids of various optical absorbance (opacity). The surviving phage were cultured and counted after irradiation, and the log reduction in numbers used to calculate the Fluence.

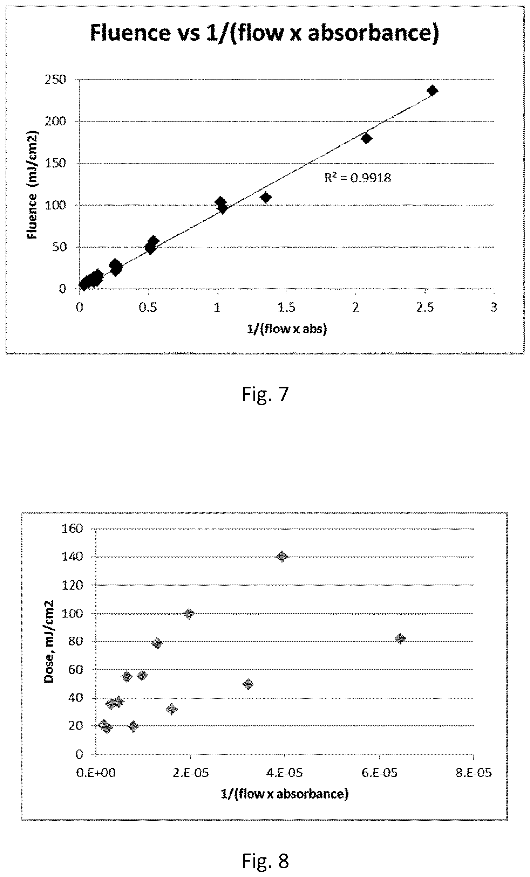

It was found that the reactor behaved in an ideal fashion, with measured Fluence inversely proportional to flow rate and absorbance - see Figure 7.

Thin film reactors are often used to treat strongly absorbing fluids, depending on the thin water layer to induce good mixing. A very thin film reactor, with a 1mm annular flow layer, was built around the same lamp as that used in the inventive reactor. This reactor was tested using the same organisms and range of fluid optics used in the test of the inventive reactor, and the results are shown in the figure 8. In contrast to the inventive reactor, the data do not form a straight line in this plot, and do not even cluster on a single curve. This performance is poor, and indicates that good mixing was not attained.

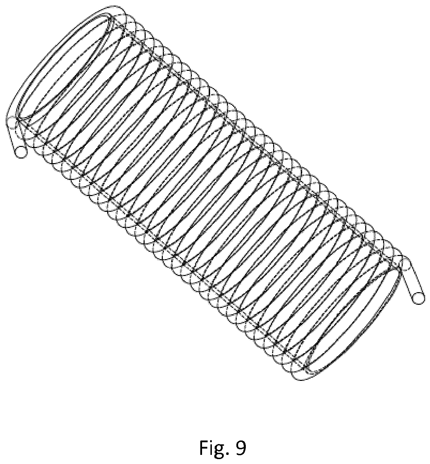

A helical spiral reactor was also constructed for comparison against the inventive reactor. The same lamp and power supply was used, and the same type and diameter of Teflon tubing was used to create a close-packed helical flow path around the central lamp, as taught by US7695675, US5069782 and US5150705. A diagram of the reactor is shown in Figure 9. Tests were conducted in the same manner as those for the other reactors, with challenge organisms being treated by pumping a test fluid of known absorbance through the helical flow path.

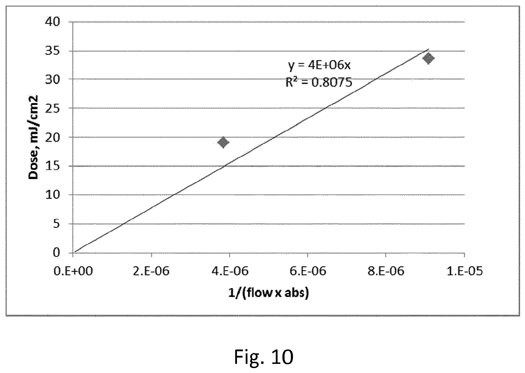

The results were plotted in similar manner and may be seen in Figure 10. Clearly the performance of this reactor does not follow the same 1/flow behavior that is achieved by the inventive reactor.

### MODELING

A variant of this reactor design, using a fluid conduit consisting of offset curved sections separated by straight sections, was modeled using Fluent CFD software. This software can simulate fluid flow by using the Navier-Stokes equations coupled with the continuity equations, and is used extensively in aerodynamic and other applications.

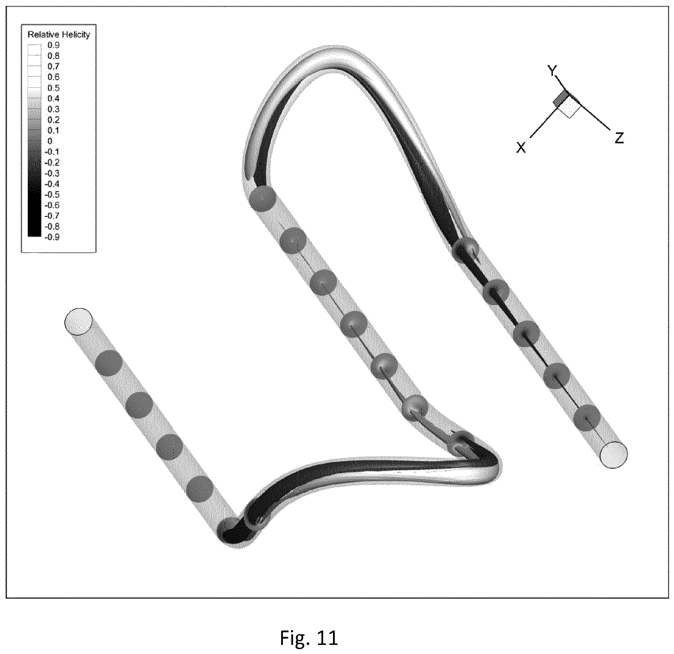

The simulated flow through the curved sections of the reactor resulted in counter-rotating vortices, as expected. The Fluent software has the capability to show the magnitude of "helicity", or rotational flow, corresponding to rotating vortices. This flow is represented schematically in Figure 11, and the simulated flow is shown in the following figure. This figure shows that the curved sections of the fluid conduit result in positive and negative helicity, shown in white or dark color, respectively, corresponding to counter rotating vortices (Dean vortices).

This reactor was also fabricated and used to inactivate MS2 phage (virus) so that the applied UV Dose could be measured. The measured results agreed very closely with the predictions from the CFD simulation, as are shown in Figure 11.

## Claims

1. A device for fluid treatment, comprising
an UV-radiation source assembly,
and a tubular fluid conduit that is at least partially transparent to radiation from the source assembly,
wherein the tubular fluid conduit is configured to have repeated patterns of four curved sections in sequence,
wherein each curved section is in a plane,
where the axes of curvature of the first and third sections are parallel to each other and orthogonal to the axes of curvature of the second and fourth sections, and the axes of curvature of the second and fourth sections are orthogonal to each other, wherein said UV-radiation source assembly is placed at one axis of curvature of one of said sections and the conduit does not enclose the UV source.

2. The device defined in claim 1, wherein the tubular conduit is circular in cross section.

3. The device defined in claim 1, wherein the UV-radiation source assembly emits in the wavelength range from 200nm to 400nm.

4. The device defined in claim 1, wherein the UV-radiation source assembly comprises a mercury-vapor lamp.

5. The device defined in claim 1, wherein the UV-radiation source assembly comprises Light Emitting Diodes.

6. The device defined in claim 1, wherein the UV-radiation source assembly has a power consumption between 5 watts and 500 watts.

## Patentansprüche

1. Vorrichtung zur Fluidbehandlung, umfassend:
eine UV-Strahlungsquellen-Baugruppe,
und eine röhrenförmige Fluidleitung, welche für Strahlung aus der Quellenbaugruppe zumindest teilweise transparent ist,
wobei die röhrenförmige Fluidleitung so konfiguriert ist, dass sie sich wiederholende Strukturen von vier aufeinanderfolgenden gekrümmten Abschnitten aufweist,
wobei sich jeder gekrümmte Abschnitt in einer Ebene befindet,
wobei die Krümmungsachsen des ersten und des dritten Abschnitts parallel zueinander und orthogonal zu den Krümmungsachsen des zweiten und des vierten Abschnitts verlaufen und die Krümmungsachsen des zweiten und des vierten Abschnitts orthogonal zueinander verlaufen, wobei die UV-Strahlungsquellen-Baugruppe an einer Krümmungsachse eines der Abschnitte angeordnet ist und die Leitung die UV-Quelle nicht umschließt.

2. Vorrichtung nach Anspruch 1, wobei die röhrenförmige Leitung einen kreisförmigen Querschnitt aufweist.

3. Vorrichtung nach Anspruch 1, wobei die UV-Strahlungsquellen-Baugruppe im Wellenlängenbereich von 200 nm bis 400 nm emittiert.

4. Vorrichtung nach Anspruch 1, wobei die UV-Strahlungsquellen-Baugruppe eine Quecksilberdampflampe umfasst.

5. Vorrichtung nach Anspruch 1, wobei die UV-Strahlungsquellen-Baugruppe Leuchtdioden umfasst.

6. Vorrichtung nach Anspruch 1, wobei die UV-Strahlungsquellen-Baugruppe eine Leistung von 5 Watt bis 500 Watt aufweist.

## Revendications

1. Dispositif pour le traitement des fluides, comprenant
un ensemble de source de rayonnement UV,
et un conduit tubulaire de fluide qui est au moins partiellement transparent au rayonnement provenant de l'ensemble source,
dans lequel le conduit tubulaire de fluide est configuré pour avoir des motifs répétés de quatre sections incurvées en séquence,
dans lequel chaque section incurvée est dans un plan,
où les axes de courbure des première et troisième sections sont parallèles l'un à l'autre et orthogonaux aux axes de courbure des deuxième et quatrième sections, et les axes de courbure des deuxième et quatrième sections sont orthogonaux l'un à l'autre, dans lequel ledit ensemble source de rayonnement UV est placé au niveau d'un axe de courbure de l'une desdites sections et le conduit n'enferme pas la source UV.

2. Dispositif défini dans la revendication 1, dans lequel le conduit tubulaire a une section transversale circulaire.

3. Dispositif défini dans la revendication 1, dans lequel l'ensemble de source de rayonnement UV émet dans la gamme de longueur d'onde de 200nm à 400nm.

4. Dispositif défini dans la revendication 1, dans lequel l'ensemble de source de rayonnement UV comprend une lampe à vapeur de mercure.

5. Dispositif défini dans la revendication 1, dans lequel l'ensemble de source de rayonnement UV comprend des diodes électroluminescentes.

6. Dispositif défini dans la revendication 1, dans lequel l'ensemble de source de rayonnement UV a une consommation d'énergie comprise entre 5 watts et 500 watts.
